# EUROPEAN PATENT SPECIFICATION

(11) **EP 2 137 513 B1**
(45) Date of publication and mention of the grant of the patent: **26.12.2018**
(21) Application number: 08741806.7
(22) Date of filing: 06.03.2008
(51) Int. Cl.: G01N 33/15, G01N 21/17, G01N 1/38, A61K 31/197, G01N 13/00

(54) **METHOD OF IDENTIFICATION TEST IN THE PROCESS OF QUALITY CONTROL OF THE MEDICINE "GLYCINE TABLETS FOR SUBLINGUAL APPLYING 0,1G"**
VERFAHREN FÜR IDENTIFIKATIONSTESTS BEI DER QUALITÄTSSTEUERUNG MEDIZINISCHER GLYCIN-TABLETTEN ZUR SUBLINGUALEN VERABREICHUNG
PROCÉDÉ DE TEST D'IDENTIFICATION LORS DU PROCESSUS DE CONTRÔLE QUALITÉ DE MÉDICAMENTS DE TYPE "PASTILLES DE GLYCINE POUR APPLICATION SUBLINGUALE 0,1 G"

(30) Priority: 07.03.2007 EA 200700597
(43) Date of publication of application: 30.12.2009
(73) Proprietor: Chastnoe Uchrezhdenie "Nauchno-Issledovateljskiyj Institut Citokhimii I Molekulyarnoyj Farmakologii", Moscow 115404 (RU)
(72) Inventor: KOMISSAROVA, Irina Alekseevna, Moscow, 115304 (RU); SOLADATENKOVA, Tatjyana Dmitrievna, Moscow, 109028 (RU); GUDKOVA, Yuliya Vasiljevna, Zelenograd, 124482 (RU); KONDRASHOVA, Tatjyana Tikhonovna, Moskow, 127224 (RU); BURBENSKAYA, Nataljya Mikhayjlovna, Moscow, 115569 (RU)
(74) Representative: Käosaar, Jüri
(86) International application number: PCT/RU2008/000130
(87) International publication number: WO 2008/108689

(56) References cited:
- WO-A1-2007/075124
- WO-A2-2004/078131
- EA-B1- 007 615
- RU-C1- 2 012 869
- SU-A1- 1 361 477
- US-A1- 2002 004 244
- 'Gosudarstvennaya farmakopeya SSSR. Desyatoe izdanie' MEDITSINA, MOSKVA 1968, pages 667 - 669, XP008143769

## Description

### Field of application

The invention relates to the chemical-pharmaceutical industry and specifically to mix for identification test in the process of quality control of the medicine "Glycine tablets for sublingual applying 0,1g", methods of its preparation and identity evaluation during quality control of the specified medicine.

### Background of invention

The medicine "Glycine tablets for sublingual applying 0,1g" (hereinafter referred to as Glycine) activates inhibitory processes in central nervous system, exhibits properties of α₁ adrenoceptor blocking agent and has stressprotecive, antistress, nootropic and neuroprotecrive effect.

For production of the medicine "Glycine tablets for sublingual applying 0,1g" there are used microcapsules in the form of non-agglomerated crystals of amino-acetic acid filmed by water-soluble methyl-cellulose, MC-100 and magnesium stearate.

Long-term experience of Glycine utilization has demonstrated that the optimum efficacy is achieved by a tablet with the decomposition period varying from 10 to 20 minutes, crushing resistance ranging from 10 to 30 N, containing 0,101g of microcapsuled glycine and 0,001g of magnesium stearate.

Dissolution of the medicine with the specified composition and physico-chemical properties is attended by buildup of special structures which provide maximum therapeutic effect. In its turn character and degree of structuring by using particular methods render it possible to carry out quantitative assessment of tablet qualitative composition in aqueous medium. (EA B1 Nº 7615, IPC 8 G01N 21/17, 2006, WO2007/75124, that is equivalent to EA B1 7615).).

In the process of manufacturing of the medicine Glycine, taking into consideration formulation and low content of adjuncts most difficulties occur with adherence to the interval of decomposition period since amino-acetic acid is easily *water*-soluble. It is easy to overcome these difficulties if pelletizing mass formulation is adhered to. Glycine plays part of a special ingredient structure in the form of microcapsules (non-agglomerated crystals of amino-acetic acid, filmed by water-soluble methyl-cellulose MC-100) in this formulation whereas a tablet itself as a 'substance' is composition structure, obtained by compression which save to the utmost microcapsules integrity.

Controllable decomposition period can be attained by use of microcapsuled glycine and strict adherence to the particular pelletizing mass formulation. (RU C1 Nº2171673, IPC 7A61K9/50,2001).

Preparation of the medicine "Glycine tablets for sublingual applying 0,1g" of high efficiency requires strict adherence to qualitative and quantitative formulation, use of the particular ingredient structure (microcapsuled Glycine), included in palletizing mass and particular structure of final substance - medicine, that can be achieved upon condition that high production standards are observed.

Taking into account some difficulties in production of the drug "Glycine tablets for sublingual applying 0,1g" (as related to decomposition period), lack of simple and accessible methods of identification of adjunct substances in the State quality standard of drugs leads to massive violations of the medicine formulation concerning adjunct substances by unscrupulous manufacturers for the purpose of frivolous cheapening. Such violations mostly result in self-tapering action and in some cases loss of its pharmacological effects. In this context it is worthy of note that 140 years after amino-acetic acid had been invented pharmacological effects were obtained only with the particular medicine structure and method of its utilization.

There is known mix for identity test of the medicine "Glycine tablets for sublingual applying 0,1g", involving purified water and porphyrized tablets in a ratio 100:1 with light transmission coefficient of 4 ml mix and layer thickness of 10mm and wave length 700±2nm in comparison with purified water ranging from 50% to 70%.( pharmacopeia FC 42-0159-05; EA B1 Nº7615, IPC 8 GO1N21/17,2006).

It has been shown practically that the base mix specified makes us evaluate violations of the formulation identity for water-soluble and non-soluble substances. Adjuncts abundance and manufacturers sophistication referring to formulation violation require extra methods of evaluation for adulteration detecting. In the US2002/0004244 there is described the use of co-solvent for preparation of new solution for the dissolving of the compound. Suitable co-solvents may be selected from water-miscible organic solvents, such as acetonitrile, methanol, ethanol, isopropanol, 1-propanol, ethylene glycol, propylene glycol 400 (PEG-400), 1,4-dioxane, dimethylformamide, acetone, tetrahydrofuran, dimethyl-sulfoxide (DMSO), and mixtures thereof.

WO2004/078131 relates to screening of multiple drug compositions for stability and compatibility, when composed to various conditions over time. The samples are prepared to provide solid drug candidates in solution form (WO2004/078131, [0099]). As co-solvents can be used ethanol, propylene glycol and glycerin. As suitable solvents, that can be used, are mentioned acetone, ethanol, benzyl benzoate, corn oil, cottonseed oil, ethyl acetate, glycerin, peanut oil, acetonitrile, tetrahydrofuran, dimethylsulfoxide and sesame oil.

### Substance of invention

The present invention is aimed at elaboration of a method as defined in claim 1 of identity test in the process of quality control of the medicine "Glycine tablets for sublingual applying 0,1g", a metabolic, with stressprotective, antistress, nootropic and neuroprotective effect for single or longterm administration, containing glycine 0,101g in the form of microcapsules (non-agglomerated crystals filmed by water-soluble methylcellulose, MC-100 or in the form of granulates and magnesium stearate (0.001g.) with decomposition period varying from 10 to 30 minutes and crush resistance from 10 to 30 N.

There is set the task of composition identity evaluation which is usually achieved by qualitative and quantitative determination of each component, i.e. the task of quantitative examination (evaluation) of tablet qualitative composition, taking into account the interaction character of medicinal substance and adjunct components in hydro-alcohol medium (50% ethanol) and furthermore in comparison to indicators of quantitative evaluation of tablet qualitative composition and the way of interaction of the medicine and adjunct substances in aqueous medium inclusively.

New quality control means must complete the known quality control methods of "Glycine tablets for sublingual applying 0.1g." already applied which together with new means must demonstrate that they meet medicine quality requirements providing its optimum therapeutic efficacy.

The given tasks are being accomplished in the following way: there is prepared mix for identity check of the medicine "Glycine tablets for sublingual applying 0,1g" involving 50% ethanol and porphyrized tablets "Glycine tablets for sublingual applying 0,1g" in the ratio 100:0.5.

Mix preparation method comprises dissolving of 1.25g of porphyrized tablets in 250 ml of 50% ethanol for 20 minutes at 40°C in an apparatus for dissolving determination at a paddle rotation speed of 200 rpm. Then mix is allowed for 10 minutes RT (room temperature).

Identification test in the process of quality control of the medicine "Glycine tablets for sublingual applying 0,1g" involves hydro-alcohol mix preparation using of 50% ethanol as described afore. Then 4ml of the mix are selected and measured on spectrophotometer for light transmission at a wave length 700±2nm in a cuvet with layer thickness of 10mm relative to 50% ethanol. Water mix is prepared by dissolving of 2.5g of porphyrized tablets in 250ml of purified water for 20 minutes at 37°C in the apparatus for dissolving determination at a paddle rotation speed of 150 rpm. Then mix is allowed for 10 minutes, 4ml of mix selected are subjected to spectrophotometer examination in a cuvet with layer thickness of 10mm versus purified water at a wave length of 700±2mm. Later on there is determined difference between light transmission coefficients of hydro-alcohol mix and water mix and the result obtained is compared to the limit of 30% to 50%.

The mix and techniques described aid to test efficiently identity of the medicine "Glycine tablets for sublingual applying 0,1g" since adjuncts not specified by the actual formulation, are water- or alcohol-soluble that is different from the solubility of the actual components. Adjuncts added by unscrupulous manufacturers for the purpose to get the required decomposition period are easy water-soluble and endowed with bounding effect, increase light transmission value up to 70-90% (instead of 50-70%). Addition of non-water-soluble adjuncts (e.g. talc, chalk etc) with different alcohol-solubility degree is defined due to mix investigation on base of 50% ethanol with further estimation of difference in light transmission values of hydro-alcohol and water mixes.

### Realization of invention

Quality control of the medicine "Glycine tablets for sublingual applying 0.1g." for each batch is carried out in laboratories of quality control departments in pharmaceutical companies and in test laboratories of certification centres.

Hydro-alcohol and water mixes are prepared simultaneously and consistently.

Hydro-alcohol mix is prepared by dissolving of 1.25g (precision weight) of porphyrized tablets in 250ml of 50% ethanol for 20 minutes at a temperature of 40°C in the apparatus for dissolving determination, paddle "Erweka" at a paddle rotation speed of 200 rpm. Then mix is allowed for 10 minutes RT and 4ml of the mix selected are subjected to spectrophotometer examination at a wave length of 700±2nm in a cuvet with layer thickness of 10mm versus 50% ethanol.

Water mix is prepared by dissolving of 2.5g of porphyrized tablets in 250ml of purified water for 20 minutes at 37°C in the apparatus for dissolving determination, paddle "Erweka" at a paddle rotation speed of 150 rpm. Then mix is allowed for 10 minutes RT, 4ml of mix selected are subjected to spectrophotometer examination in a cuvet with layer thickness of 10mm versus purified water at a wave length of 700±2nm.

Light transmission coefficient of hydro-alcohol mix must lie within the limits (90-100%). Light transmission coefficient of water mix must vary from 50% to 70%.

There is determined difference between light transmission coefficients of hydro-alcohol and water mixes. The value obtained ranging from 30% to 50% corresponds to appropriate composition and quality of the medicine "Glycine tablets for sublingual applying 0.1g." endowed with the necessary therapeutic effect.

Confirmation of interaction of the "Glycine tablets for sublingual applying 0.1g." determinate quantity and quality content and light transmission values is demonstrated in the table presenting the analysis results made in accordance with the below examples.

### Example 1

1.25 g. of the porphyrized "Glycine tablets for sublingual applying 0.1g." were dissolved in 250ml of 50% ethanol for 20 minutes at 40°C in the apparatus for dissolving determination, paddle "Erweka" at a paddle rotation speed of 200 rpm.

Decomposition period of tablets with mottle elements varied from 10 to 20 minutes, resistance ranged from 10 to 30N. The tablets contained 0.101g. of microcapsulated glycine and 0.001g of magnesium stearate.

On complete dissolution of the tablets obtained after porphyrizing within 20 minutes, mix was allowed for 10 minutes RT, then there were selected 4ml of mix in a cuvet with layer thickness of 10mm and later subjected to spectrophotometer examination versus solvent (50% ethanol) at a wave length of 700±2nm.

Light transmission coefficient was 98.6%.

2.5g of the porphyrized "Glycine tablets for sublingual applying 0.1g." (formulation and physico-chemical properties were mentioned before) were dissolved in 250ml of water for 20 minutes at 37°C in the apparatus for dissolving determination, paddle "Erweka" at a paddle rotation speed of 150 rpm.

On complete dissolution of powder within 20 minutes, mix was allowed for 10 minutes RT, then there were selected 4ml of mix in a cuvet with layer thickness of 10mm and later subjected to spectrophotometer examination versus solvent (purified water) at a wave length of 700±2nm.

Light transmission coefficient was 63%.

Difference in light transmission coefficients of hydro-alcohol and water mixes is 35.6%.

In the similar way there were prepared mixes with tablets containing amino-acetic acid 0.1g, water-soluble methylcellulose, MC-100 - 0.001g in granulates and magnesium stearate 0.001g. in the function of powder.

The results are presented in the table below.

### Example 2

1.25 g. of the porphyrized tablets containing 0.1g of amino-acetic acid, 0.001g of water-soluble methylcellulose, MC-100 and 0.001g calcium stearate, were dissolved in 250ml of 50% ethanol according the run of example 1.

Light transmission coefficient was 63%.

2.5g of the porphyrized tablets containing 0.1g of amino-acetic acid, 0.001g of water-soluble methylcellulose, MC-100 and 0.001g of calcium stearate, were dissolved in 250ml of water according the run of example 1.

Light transmission coefficient was 62%.

Difference in light transmission coefficients of hydro-alcohol and water mixes is 1%.

### Example 3

1.25 g. of the porphyrized tablets containing 0.1g of amino-acetic acid, 0.001g of water-soluble methylcellulose, MC-100, 0.0005g of magnesium stearate and 0.0005g of calcium stearate, were dissolved in 250ml of 50% ethanol according the run of examples 1 and 2.

Light transmission coefficient was 70%.

2.5g of the porphyrized tablets containing 0.1g of amino-acetic acid, 0.001g of water-soluble methylcellulose, MC-100, 0.0005g of magnesium stearate and 0.0005g

of calcium stearate, were dissolved in 250ml of water according the run of examples 1 and 2.

Light transmission coefficient was 62%.

Difference in light transmission coefficients of hydro-alcohol and water mixes was 8%.

### Example 4

In order to obtain hydro-alcohol and water mixes there were used tablets containing 0.1g of amino-acetic acid, 0.0005g of water-soluble methylcellulose, MC-100, and 0.0005g of magnesium stearate.

All investigations were carried our in a way similar to examples 1 and 2.

Light transmission coefficient of hydro-alcohol mix was 99%.

Light transmission coefficient of water mix was 79%.

Difference amounted to 20%,

### Example 5

In order to obtain hydro-alcohol and water mixes there were used tablets (the medicine Glicised - KMP batches 14.08.05,15.09.05), involving 0.1g of amino-acetic acid and adjunct components in unknown quantities: oydragit RS-30D, montaniac glycolic wax, polyvinylpirrolidone lowmolecular medicinal and calcium stearate. Experiments were held according to the run of examples 1 and 2.

Light transmission coefficient of hydro-alcohol mix corresponded to 74.7% and 72.7%.

Light transmission coefficient of water mix corresponded to 55% and 59%.

Difference amounted to 19.7%, 13.2%.

### Example 6

In order to obtain hydro-alcohol and water mixes there were used tablets (Gromecine batches 05.07.06, 07.07.06), involving 0.1g of amino-acetic acid, 0.001g of water-soluble methylcellulose, MC-100, and 0.001g of magnesium stearate, in accordance with the instruction. Experiments were held according to the run of examples 1 and 2.

Light transmission coefficient of hydro-alcohol mix was 97.1 %, 98.3%.

Light transmission coefficient of water mix was 76.9%, 90.1%.

Difference - 20.2%, 8.2%.

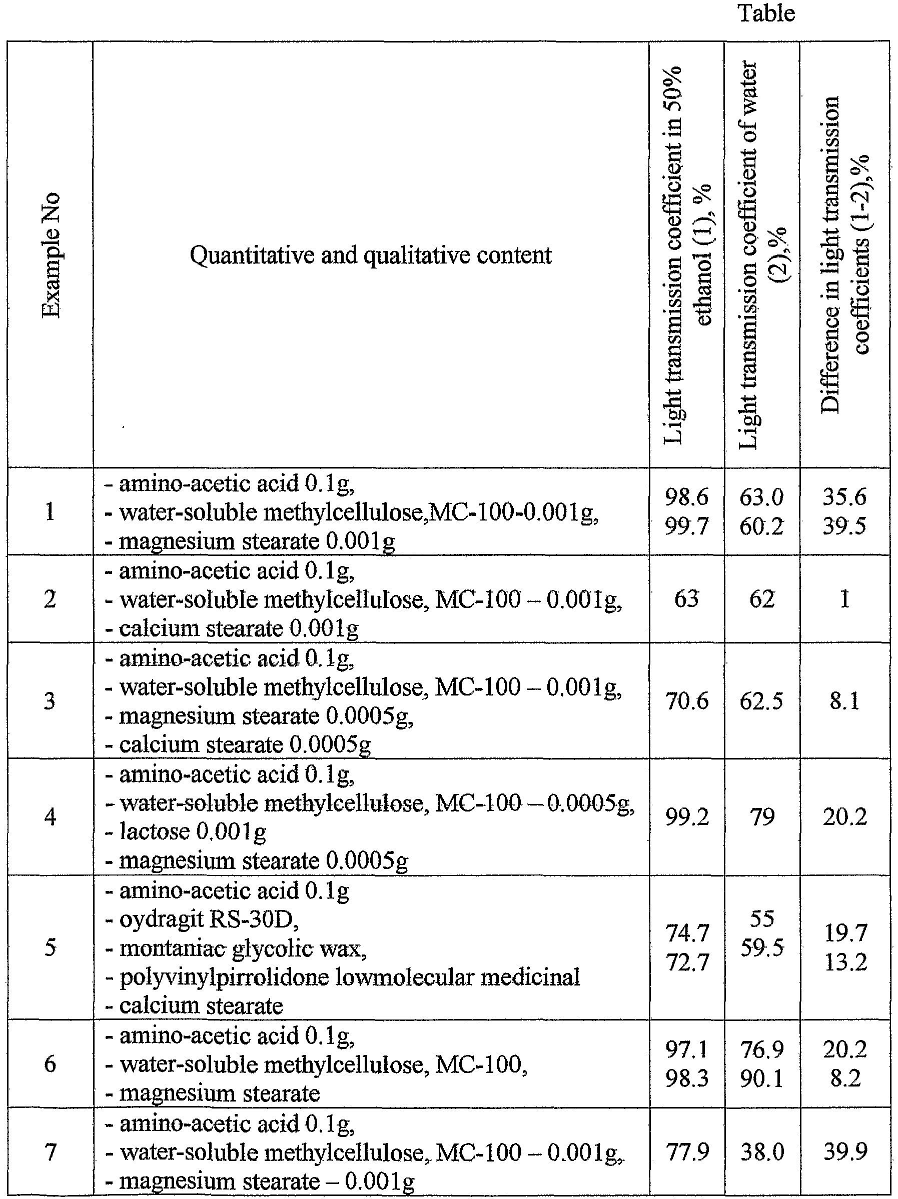

### Example 7

In order to obtain hydro-alcohol and water mixes there were used tablets (produced by company OOO "Vicher-Pharm"), involving 0.1g of amino-acetic acid, 0.001g of water-soluble methylcellulose, MC-100, and 0.001g of magnesium stearate, batch 03.03.04 in accordance with the instruction. Experiments were held according to the run of examples 1 and 2.

Light transmission coefficient of hydro-alcohol mix was 77.9%.

Light transmission coefficient of water mix was 38.0%.

Difference - 39.9%.

## Claims

1. Method of identification test in the process of quality control of the medicine "Glycine tablets for sublingual application 0.1 g", containing 0.1 g of amino-acetic acid and 0.001 g of water-soluble methylcellulose, MC-100 in the form of microcapsules of non-agglomerated amino-acetic acid crystals covered with a polymeric film of water-soluble methylcellulose, MC-100 or in the form of granulates and 0.001 g of magnesium stearate, said tablets having a decomposition period varying from 10 to 20 minutes and a crushing resistance ranging from 10 to 30 N, wherein said method comprises
- preparing an hydro-alcohol mix by dissolving of 1.25 g of porphyrized tablets in 250 ml of an hydro-alcohol solvent containing 50 % ethanol for 20 minutes at temperature of 40 °C in a dissolution apparatus at a paddle rotation speed of 200 rpm; allowing the obtained mix for 10 minutes at room temperature; selecting 4 ml of the mix in a cuvette with layer thickness of 10 mm; examining said mix in the cuvette with a light transmission spectrophotometer at a wave length of 700±2 nanometers; determining whether the light transmission coefficient versus the hydro-alcohol solvent containing 50 % ethanol is within the limits of 90 to 100 %;
- preparing a water mix by dissolving of 2,5 g of porphyrized tablets in 250 ml of purified water for 20 minutes at a temperature of 37 °C in a dissolution apparatus at a paddle rotation speed of 150 rpm, allowing the obtained mix for 10 minutes at room temperature, selecting 4 ml of the mix in a cuvette with layer thickness of 10 mm; examining said mix in the cuvette with a light transmission spectrophotometer at a wave length of 700±2 nanometers; determining whether the light transmission coefficient versus the purified water is within the limits of 50 to 70 %;
determining whether the difference between the light transmission coefficients of the hydro-alcohol mix and of the water mix is within the limit of 30 to 50 %.

## Patentansprüche

1. Verfahren zum Bestimmungstest bei der Durchführung der Qualitätskontrolle des Arzneimittels "0,1g Glycin-Tabletten zur Anwendung unter der Zunge", bestehend aus 0,1g Aminoessigsäure und 0,001g wasserlöslicher Methylcellulose, MC-100 in Form von Mikrokapseln nicht agglomerierter Aminoessigsäurekristalle umhüllt mit einem fünffachen Polymerfilm wasserlöslicher Methylcellulose, MC-100 oder in Form von Granulaten und 0,001g Magnesiumstearat, wobei die besagten Tabletten einen Zersetzungszeitraum von 10 bis 20 Minuten und eine Druckfestigkeit zwischen 10N und 30N besitzen und das besagte Verfahren beinhaltet
- Vorbereitung einer Wasser-Alkohol-Mischung durch Lösung von 1,25g porphyrierter Tabletten in 250ml einer Wasser-Alkohol-Lösung bestehend aus 50% Ethanol für 20 Minuten bei einer Temperatur von 40°C in einer Lösungsvorrichtung bei einer Drehbewegungsgeschwindigkeit von 200 U/min; Beibehaltung der erhaltenen Mischung für 10 Minuten bei Zimmertemperatur; Auswahl von 4ml der Mischung mittels einer Küvette mit Schichtdicke von 10mm; Untersuchung besagter Mischung in der Küvette mit einem lichtübertragenden Spektrofotometer bei einer Wellenlänge von 700±2 Nanometern; Bestimmung ob der Lichtübertragungskoeffizient gegenüber der Wasser-Alkohol-Lösung 50% Ethanol enthält und sich innerhalb der Grenzwerte von 90 bis 100% befindet;
- Vorbereitung einer Wassermischung durch Lösung von 2,5g porphyrierter Tabletten in 250ml destilliertem Wasser für 20 Minuten bei einer Temperatur von 37°C in einer Lösungsvorrichtung bei einer Drehgeschwindigkeit von 150 U/min; Beibehaltung der Mischung für 10 Minuten bei Zimmertemperatur, Auswahl von 4ml der Mischung mittels einer Küvette mit Schichtdicke von 10mm; Untersuchung besagter Mischung in der Küvette mit einem lichtübertragenden Spektrofotometer bei einer Wellenlänge von 700±2 Nanometern; Bestimmung ob der Lichtübertragungskoeffizient sich gegenüber dem destillierten Wasser innerhalb der Grenzwerte von 50 bis 70% befindet;
Bestimmung ob sich die Differenz zwischen dem Lichtübertragungskoeffizienten der Wasser-Alkohol-Mischung und der Wassermischung innerhalb des Grenzwertes von 30 bis 50% befindet.

## Revendications

1. Procédé d'essai d'identification dans le processus de contrôle de qualité du médicament "Comprimés de glycine pour application sublinguale 0,1 g", contenant 0,1 g d'acide amino-acétique et 0,001 g de méthylcellulose hydrosoluble, MC-100 sous forme de microcapsules de cristaux d'acide amino-acétique non agglomérés recouverts par un film polymère de méthylcellulose hydrosoluble, MC-100 ou sous forme de granules, 0.001 g de stéarate de magnésium, lesdits comprimés ayant une période de décomposition variant de 10 à 20 minutes et une résistance à l'écrasement variant de 10 à 30 N, où ledit procédé comprend
- la préparation d'un mélange hydro-alcoolique par dissolution de 1.25 g de comprimés porphyrifiés dans 250 ml d'un solvant hydro-alcoolique contenant 50 % d'éthanol pendant 20 minutes à une température de 40 °C dans un appareil de dissolution à une vitesse de rotation de la palette de 200 tr/min; laisser le mélange obtenu pendant 10 minutes à température ambiante; sélectionner 4 ml du mélange dans une cuvette avec épaisseur de couche de 10 mm; examiner ledit mélange dans la cuvette avec un spectrophotomètre de transmission de lumière à une longueur d'onde de 700 ± 2 nanomètres; déterminer si le coefficient de transmission de lumière par rapport au solvant hydro-alcoolique contenant 50 % d'éthanol est dans les limites de 90 à 100 %;
- préparation d'un mélange d'eau par dissolution de 2,5 g de comprimés porphyriques dans 250 ml d'eau purifiée pendant 20 minutes à une température de 37 °C dans un appareil de dissolution à une vitesse de rotation de la pale de 150 tr/min, en laissant le mélange obtenu pendant 10 minutes à température ambiante 20, en sélectionnant 4 ml du mélange dans une cuvette ayant une épaisseur de couche de 10 mm; examen dudit mélange dans la cuvette avec un spectrophotomètre de transmission de lumière à une longueur d'onde de 700 ± 2 nanomètres; détermination si le coefficient de transmission de lumière par rapport à l'eau purifiée est dans les limites de 50 à 70 %;
déterminer si la différence entre les coefficients de transmission de la lumière du mélange hydro-alcoolique et du mélange d'eau se situe dans la limite de 30 à 50 %.
